(19) Europäisches Patentamt
European Patent Office
Office européen des brevets

(11) EP 2 016 933 A1

(12) **DEMANDE DE BREVET EUROPEEN**

(43) Date de publication:
**21.01.2009 Bulletin 2009/04**

(21) Numéro de dépôt: **08160223.7**

(22) Date de dépôt: **11.07.2008**

(51) Int Cl.:
***A61K 8/58*** *(2006.01)* ***A61K 8/891*** *(2006.01)*
***A61K 8/892*** *(2006.01)* ***A61Q 5/06*** *(2006.01)*
***A61Q 5/12*** *(2006.01)* ***A61K 8/19*** *(2006.01)*

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **13.07.2007 FR 0756484**

(71) Demandeur: **L'Oréal**
**75008 Paris (FR)**

(72) Inventeur: **Brun, Gaëlle**
**75015 Paris (FR)**

(74) Mandataire: **Fevrier, Murielle Françoise E.**
**L'Oreal - D.I.P.I.**
**River Plaza**
**25-29 Quai Aulagnier**
**92665 Asnières-sur-Seine (FR)**

(54) **Composition cosmétique anhydre comprenant un copolymère silicone adhésif sensible à la pression, une silicone volatile et une silicone fluide particulière**

(57) La présente invention a pour objet une composition de traitement des cheveux anhydre qui comprend un ou plusieurs copolymères à base de résine de silicone et de silicone fluide, une ou plusieurs silicones volatiles linéaires ou cycliques, et une ou plusieurs polydiméthylsiloxane linéaire non volatile de viscosité supérieure à 5 cSt, la quantité de copolymère étant supérieure à 1 % en poids du poids total de la composition et la composition étant exempte de pigments ou contenant plus de 5 % de pigments.

On obtient ainsi sur les fibres kératiniques des gainages permettant d'obtenir du volume, de la masse et du corps aux cheveux de façon rémanente aux shampooings tout en préservant les qualités physiques de la fibre kératiniques, notamment des gainages colorés.



Printed by Jouve, 75001 PARIS (FR)

## Description

La présente invention a pour objet une composition de traitement des fibres kératiniques notamment les cheveux ainsi qu'un procédé de traitement des fibres kératiniques mettant en oeuvre ces compositions.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Cette dégradation des cheveux est par ailleurs accrue par la répétition de traitement de coloration permanente des cheveux, qui consiste à appliquer sur les cheveux un précurseur de colorant et un agent oxydant.

Ainsi, pour remédier à cela, il est maintenant usuel d'utiliser des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner.

Un inconvénient lié à l'utilisation de ces compositions capillaires réside dans le fait que les effets cosmétiques conférés par de telles compositions ont tendance à disparaître, notamment dès le premier shampooing. Ceci est d'autant plus vrai lorsqu'un des effets conférés est un effet coloriel apporté par des pigments.

Afin de remédier à cet inconvénient, il est envisageable d'accroître la rémanence du dépôt de polymères en effectuant directement une polymérisation radicalaire de certains monomères au niveau des cheveux. Toutefois, les traitements ainsi obtenus entraînent une dégradation de la fibre et les cheveux ainsi traités sont généralement difficilement démélables.

Il est par ailleurs connu d'effectuer des gainages des cheveux à partir d'une composition comprenant un monomère électrophile de type cyanoacrylate, notamment dans les demandes de brevet FR 2 833 489. Une telle composition permet d'obtenir des cheveux parfaitement gainés et non gras. Cependant, le gainage obtenu necessite des conditions opératoires particulières dues à la réactivité du monomère électrophile. Par ailleurs le gainage obtenu avec ces monomères électrophiles devient collant avec les corps gras tels que le sébum.

Il existe aussi des copolymères de silicone particuliers qui comprennent un segment résine de silicone et un segment de silicone fluide, plus connus sous le nom de BioPSA. Ces copolymères sont notamment décrits dans WO 03/026596, WO 2004/073626, WO 2007/051505 et WO 2007/051506 pour diverses applications cosmétiques telles que l'application sur les cheveux, les ongles, la peau.

Ainsi, le but de la présente invention est de mettre au point un procédé de traitement des fibres kératiniques telles que les cheveux facile de mise en oeuvre qui permet d'obtenir des gainages rémanents aux shampoings et aux diverses agressions que peuvent subir les cheveux, notamment les brushings et la transpiration tout en tolérant mieux les corps gras tels que le sébum. Notamment, le but de la présente invention est d'obtenir des gainages colorés, faciles à mettre en oeuvre, résistants aux agents extérieurs et respectant l'intégrité des fibres kératiniques.

Ce but est atteint avec la présente invention qui a pour objet une composition anhydre de traitement des cheveux qui comprend un ou plusieurs copolymères à base de résine de silicone et de silicone fluide, une ou plusieurs silicones volatiles linéaires ou cycliques, et une ou plusieurs polydiméthylsiloxane linéaire non volatile de viscosité supérieure à 5 cSt, la quantité de copolymère étant supérieure à 1 % en poids du poids total de la composition et la composition étant exempte de pigments.

La composition de l'invention a aussi pour objet une composition de coloration des fibres kératiniques anhydre comprenant un ou plusieurs copolymères à base de résine de silicone et de silicone fluide, une ou plusieurs silicones volatiles linéaires ou cycliques, une ou plusieurs polydiméthylsiloxane linéaire non volatile de viscosité supérieure à 5 cSt, un pigment colorant, la quantité de copolymère étant supérieure à 1 % en poids du poids total de la composition et la quantité de pigments colorants étant supérieure à 5 %.

L'invention a aussi pour objet un procédé de traitement des fibres kératiniques pour l'obtention d'un effet rémanent aux shampoings mettant en oeuvre l'une ou l'autre de ces compositions.

Un autre objet de l'invention est l'utilisation d'une composition anhydre comprenant un ou plusieurs copolymères à base de résine de silicone et de silicone fluide avec un ou plusieurs pigments colorants pour l'obtention de gainage coloré ou sans pigment pour obtenir un simple gainage.

On obtient ainsi sur les fibres kératiniques des gainages permettant d'obtenir du volume, de la masse et du corps aux cheveux de façon rémanente aux shampooings tout en préservant les qualités physiques de la fibre kératiniques, avec de plus un effet coloriel rémanent lorsque la composition contient des pigments. Un tel gainage est en particulier résistant aux agressions extérieures que peuvent subir les cheveux telles que le brushing et la transpiration. Il permer de plus d'obtenir des colorations permanentes sans utilisation d'agents oxydants susceptibles de dégrader les cheveux.

Le gainage ainsi formé se présente sous forme d'un dépôt homogène, lisse et possède une excellente adhésion sur cheveux. Par ailleurs, on a constaté de façon surprenante que les cheveux restaient parfaitement individualisés, pouvaient être coiffés sans problème et que les propriétés de coiffant apportées à la fibre étaient rémanentes aux shampooings.

***Copolymère à base de résine de silicone et de silicone fluide***

Le copolymère siliconé défini selon l'invention est dérivé de la réaction entre une résine de silicone et une silicone fluide.

De tels copolymères sont décrits par exemple dans « Silicone Pressure Sensitive Adhesive », Sobieski and Tangney, Handbook of Pressure Sensitive Adhesive Technology (D. Satas Ed.), Von Nostrand Reinhold, New York.

Dans le copolymère, la résine de silicone est présente à un taux compris entre 45 et 75% (par rapport à la masse totale de silicone) et la silicone fluide est présente à un taux compris entre 25 et 55%, avec la somme des pourcentages de résine de silicone et de silicone fluide qui est égal à 100. De préférence, la résine de silicone est présente à un taux compris entre 55 et 65% (par rapport à la masse totale de silicone) et la silicone fluide est présente à un taux compris entre 35 et 45%, avec la somme des pourcentages de résine de silicone et de silicone fluide qui est égal à 100.

De préférence, la résine de silicone selon l'invention est le produit de condensation de groupements $SiO_2$ et de groupements $R_3(SiO)_{1/2}$ (triorganosilyl) pour lequel chaque groupement R est indépendamment sélectionné parmi les radicaux méthyle, éthyle, propyle ou vinyl et pour lequel le rapport entre les fonctions $SiO_2$ et les fonctions $R_3(SiO)_{1/2}$ de la résine de silicone va de 0.6 à 0.9. Des groupements triorganosilyl utilisables pour former la résine de silicone peuvent être des unités triméthylsilyl, triéthylsilyl, méthylméthylproprylsilyl, diméthylvinylsilyl et des mélanges de celles-ci. Le groupement triméthylsilyl est le préféré dans le cadre de l'invention.

De préférence, la silicone fluide selon l'invention est une diorganopolysiloxane aux fonctions OH terminales ayant une viscosité comprise entre 100 et 100.000 cst à 25°C pour laquelle les substituants du diorganopolysiloxane sont indépendamment choisis parmi les radicaux méthyle, éthyle, propyle ou vinyl. Les diorganosiloxances sont de préférence des polymères linéaires. Des exemples de diorganopolysiloxane peuvent être, de manière non limitative, une polydiméthylsiloxane, une éthylméthylpolysiloxane, un copolymère de diméthylsiloxane et de méthylvinylsiloxane, et des mélanges de tels polymères ou copolymères ayant des extrémités OH. Le diorganopolysiloxane préféré est une polydiméthylsiloxane.

Des exemples de synthèse d'un tel copolymère sont par exemple décrits dans le brevet US5162410 ou dans le brevet CA711756.

Les copolymères selon la présente invention peuvent donc être préparés en chauffant le mélange suivant :

1. de 45% à 75% en masse de résine de silicone étant le produit de condensation des unités $SiO_2$ et $R_3(SiO)_{1/2}$ pour lequel chaque groupement R est indépendamment sélectionné parmi les radicaux méthyle, éthyle, propyle ou vinyl et pour lequel le rapport entre les fonctions $SiO_2$ et les fonctions $R_3(SiO)_{1/2}$ de la résine de silicone va de 0.6 à 0.9.

2. de 25% à 55% en masse de diorganopolysiloxane fluide aux fonctions OH terminales ayant une viscosité comprise entre 100 et 100.000 cst à 25°C pour laquelle les substituants du diorganopolysiloxane sont indépendamment choisis parmi les radicaux méthyle, éthyle, propyle ou vinyl.

3. de 0.001% à 5% d'un catalyseur adapté, qui est de préférence un composé aminé aliphatique organique choisi de préférence parmi les amines primaires, les amines secondaires, les amines tertiaires, les sels d'acide carboxylique des amines citées ci-dessus et les sels d'ammonium quaternaires.

Le mélange est chauffé à une température comprise entre 80°C et 160°C jusqu'à ce que le caractère adhésif du copolymère siliconé résultant soit obtenu.

Les copolymères préférés selon l'invention sont commercialisés par Dow Corning sous la référence BIO-PSA®, ces BIO-PSA® pouvant être sous deux formes, standard ou amine compatible, et étant fournis dans différents solvants avec plusieurs ratio résine de silicone/silicone fluide. On peut notamment citer les grades 7-4400, 7-4500, 7-4600. Le BIO-PSA® particulièrement préféré selon l'invention est le grade 7-4400.

Le copolymère peut être présent dans la composition selon l'invention en une teneur supérieure à 1 % et jusqu'à 40 % en poids, par rapport au poids total de la composition, de préférence allant de 1,5 % à 20 % en poids, et préférentiellement allant de 1,5 % à 15 % en poids.

***Silicone volatile***

Dans le cadre de l'invention, on entend par silicone volatile, une silicone liquide à la température ambiante (25°C) et à la pression atmosphérique présentant une pression de vapeur à 25°C supérieure à 0,1 mmHg et de préférence comprise entre 0,1 et 300 mmHg, encore plus préférentiellement entre 0.1 et 200 mmHg.
Comme silicone volatile, on peut citer les silicones linéaires ou cycliques ayant de 4 à 7 atomes de silicium, ces silicones comportant éventuellement des groupes alkyle ou alcoxy ayant de 1 à 10 atomes de carbone. Comme silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, le décaméthyltétrasiloxane et leurs mélanges.
De préférence, la silicone volatile est cyclique et choisie parmi le décaméthylcyclopentasiloxane, l'octaméthyltrisiloxane et le décaméthyltétrasiloxane.
A titre d'exemple on peut citer la décaméthylcyclopentasiloxane commercialisée sous le nom DC-245 par la société Dow Corning, l'octaméthyltrisiloxane commercialisée sous le nom DC-200 Fluid 1 cst par la société Dow Corning et le décaméthyltétrasiloxane commercialisée sous le nom DC-200 Fluid 1.5 cst par la société Dow Corning
Cette silicone volatile cyclique présente généralement une viscosité faible, par exemple une viscosité inférieure à 5 cSt à 25°C.
De préférence la silicone volatile est cyclique et est le décaméthylcyclopentasiloxane commercialisé sous le nom DC-245 par la société Dow Corning.
La silicone volatile est présente dans la composition selon l'invention en une teneur allant de 0,1 % à 99 % en poids, par rapport au poids total de la composition, de préférence allant de 1 % à 95 % en poids, et préférentiellement allant de 5 % à 90 % en poids.

***PDMS linéaire non volatile de viscosité supérieure à 5 cst***

La polydiméthylsiloxane (PDMS) linéaire non volatile de viscosité supérieure à 5 cSt est notamment une gomme de silicone ou une huile de silicone de pression de vapeur inférieure à 0,1 mm Hg à 25°C.
La PDMS linéaire non volatile de viscosité supérieure à 5 cst peut être choisie parmi les polydiméthylsiloxanes; les alkyldiméthicones; les polyphénylméthylsiloxanes tels que les phényldiméthicones, les phényltriméthicones, et les vinylméthylméthicones; ainsi que les silicones modifiés par des groupements aliphatiques et/ou aromatiques, éventuellement fluorés, ou par des groupements fonctionnels tels que des groupements hydroxyles, thiols et/ou amines.
De préférence, la viscosité des PDMS linéaires non volatiles utiles dans la présente invention est supérieure à 5 cst à 25°C. Selon un mode de réalisation particulier, cette viscosité est comprise entre 5 cst et 5.000.000 cst, de préférence entre 100 cst et 4.000.000 et encore plus préférentiellement de 5000 et 4.000.000 cst
Le poids moléculaire est généralement compris entre 500 et 800.000 g/mol, de préférence de 5000 à 700.000 g/mol et encore plus préférentiellement de 50.000 à 600.000 g/mol.
Cette PDMS linéaire peut notamment être choisie parmi les silicones de formule (II):

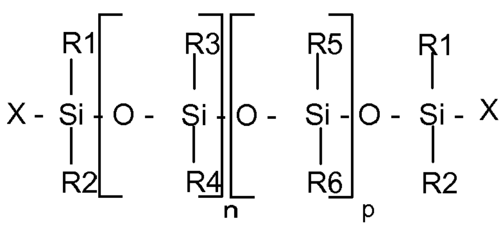

(II)

dans laquelle :

$R_1$, $R_2$, $R_5$ et $R_6$ sont, ensemble ou séparément, un radical alkyle ayant 1 à 6 atomes de carbone,

$R_3$ et $R_4$ sont, ensemble ou séparément, un radical alkyle ayant de 1 à 6 atomes de carbone, un radical vinyle ou un radical aryle,

X est un radical alkyle ayant de 1 à 6 atomes de carbone, un radical hydroxyle ou un radical vinyle, un radical amine

n et p étant des entiers choisis de manière à avoir un composé de viscosité supérieure à 5 cst, de préférence la somme n + p est supérieure à 10.

A titre d'exemple, on peut citer les polydiméthylsiloxanes suivantes :

○ les substituants $R_1$ à $R_6$ et X représentent un groupement méthyle, comme celle vendue sous la dénomination Baysilicone TP 3898 par la société Général Electric, et celle vendue sous la dénomination AK 500000 par la société Waker,

○ les substituants $R_1$ à $R_6$ et X représentent un groupement méthyle, p et n sont tels que le poids moléculaire est de 120 000 g/mol, comme celle vendue sous la dénomination Dow Corning 200 Fluid 60000 CS par la société Dow Corning.

○ les substituants $R_1$ à $R_6$ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 cst par la société Dow Corning.

○ les substituants $R_1$ à $R_6$ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning.

○ Les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

De préférence, les PDMS linéaires non volatiles ne sont oxyalkylénés.

***Pigments***

Selon une variante, la composition est une composition de coloration des fibres kératiniques qui comprend des pigments colorants. Une telle composition permet d'obtenir des gainages rémanents, colorants et ceci sans dégradation des fibres kératiniques.

Par pigment colorant, on entend tous les pigments apportant de la couleur aux matières kératiniques. Ceci exclut notamment les pigments blancs tels que le dioxyde de titane qui n'apporte que du blanc aux matières kératiniques.

Les pigments qui peuvent être utilisés sont notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome et le bleu ferrique.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

A titre d'exemple on peut aussi citer les pâtes pigmentaires de pigment organique telles que les produits vendus par la société HOECHST sous le nom :

- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica recouvert de titane, ou d'oxychlorure de bismuth, les pigments nacrés colorés tels que le mica recouvert de titane et d'oxydes de fer, Ile mica recouvert d'oxyde de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Prestige Bronze commercialisée par Eckart (Mica-Fe2O3),Colorona commercialisée par Merck (Mica-TiO2-Fe2O3).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être synthétisées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique selon la présente invention est généralement comprise entre 10 nm et 200 μm, de préférence entre 20 nm et 80 μm, et plus préférentiellement entre 30 nm et 50 μm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C8 à C20 et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments. Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :

- un traitement PEG-Silicone comme le traitement de surface AQ commercialisé par LCW ;
- un traitement Chitosane comme le traitement de surface CTS commercialisé par LCW;
- un traitement Triéthoxycaprylylsilane comme le traitement de surface AS commercialisé par LCW ;
- un traitement Méthicone comme le traitement de surface SI commercialisé par LCW;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;

- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition conforme à la présente invention peut de plus comprendre un ou plusieurs pigments non traités en surface.

Lorsqu'il est présent, la quantité de pigments peut varier jusqu'à 40 % et de préférence jusqu'à 20 %.

La composition de l'invention peut contenir d'autres espèces colorées ou colorantes telle que des colorants directs hydrophiles ou hydrophobes ou des précurseurs de colorants.

La composition de l'invention peut encore contenir d'autres constituants. Elle peut notamment comprendre un solvant organique non siliconé, volatil ou non. Le terme volatile prend la même définition que celle donnée précédemment pour les silicones volatiles

A titre de solvant organique volatil, on peut citer :

- les alcanols volatils en $C_1$-$C_4$ tels que l'éthanol, l'isopropanol;
- les alcanes volatils en $C_5$-$C_7$ tels que le n-pentane, l'hexane, le cyclopentane, le 2,3-diméthylbutane, le 2,2-diméthylbutane, le 2-méthylpentane, le 3-méthylpentane ;
- les esters d'acides en $C_1$-$C_{20}$ liquides et d'alcools en $C_1$-$C_8$ volatils tels que l'acétate de méthyle, l'acétate de n-butyle, l'acétate d'éthyle, l'acétate de propyle, l'acétate d'isopentyle, le 3-éthoxypopionate d'éthyle;
- les cétones liquides à température ambiante et volatiles telles que la méthyléthylcétone, la méthylisobutylcétone, la diisobutylcétone, l'isophorone, la cyclohexanone, l'acétone ;
- les polyols volatils tels que le propylène glycol ;
- les éthers volatils tels que le diméthoxyméthane, le diéthoxyéthane, le diéthyléther ;
- les éthers de glycol volatils comme le 2-butoxyéthanol, le butyle diglycol, le monométhyléther de diéthylène glycol, le n-butyléther de propylène glycol, l'acétate de monométhyl éther de propylène glycol.
- les huiles hydrocarbonées volatiles telles que les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, et par exemple les huiles vendues sous les noms commerciaux d'Isopars ou de Permetyls, et leurs mélanges. On peut aussi citer les neopentanoate d'isohexyle ou d'isodecyle.
- les perfluoroalcanes volatils en $C_4$-$C_{10}$ tels que dodecafluoropentane, le tetradecafluorohexane, le decafluoropentane
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine.
- les composés fluoroalkyles ou hétérofluoroalkyles volatils répondant à la formule suivante :

$$CH_3-(CH_2)_n-[Z]_t-X-CF_3$$

dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical $-(CH_2)_n-CH_3$ ou $-(CF_2)_m-CF_3$, m étant 2, 3, 4 ou 5.

Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

A titre de solvant organique non volatil, on peut citer :

- les alcools aromatiques non volatils tels que l'alcool benzylique, le phenoxyéthanol ;
- les esters d'acides en $C_1$-$C_{20}$ liquides et d'alcools en $C_1$-$C_8$ non volatils tels que le myristate d'isopropyle ;
- l'éthylène carbonate, le propylène carbonate; le butylène carbonate ;
- les polyols non volatils tels que le glycérol, l'éthylène glycol, le dipropylène glycol, le butylène glycol,
- les éthers de glycol non volatils comme le monoéhyléther de diéthylène glycol, le mono n-butyl éther de dipropylène glycol.
- les huiles hydrocarbonées non volatiles telles que l'isohexadécane.
- Les alcools gras liquides non volatils en $C_{10}$-$C_{30}$ tels que l'alcool oléique, les esters d'alcools gras en $C_{10}$-$C_{30}$ liquides tels que les benzoates d'alcool gras en $C_{10}$-$C_{30}$ et leurs mélanges ; l'huile de polybutène, l'isononanoate d'isononyle, le malate d'isostéaryle, le tétra-isostéarate de pentaérythrityle, le trimélate de tridécyle
- Les solvants perfluorés non volatils tels que le perfluoroperhydrophenanthrene, vendu sous la dénomination de "Flutec PC11®" par la Société F2 Chemicals

De préférence, le solvant organique est choisi de telle manière que son point d'ébullition soit inférieur à 200°C.

De préférence le solvant organique est un solvant organique volatil. Il est par exemple choisi parmi l'éthanol, l'isopropanol, l'acétone, l'isododécane.

Le solvant organique peut être présent dans la composition selon l'invention en une teneur allant de 0,1% à 90% en poids, par rapport au poids total de la composition, de préférence allant de 1% à 80% en poids, et préférentiellement allant de 5% à 70% en poids.

La composition de l'invention peut contenir d'autres composés siliconés particuliers autres que ceux décrits précédemment.

La composition de l'invention peut ainsi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.

Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707,EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :

a) de 0,01 à 50 % en poids d'au moins un macromère polysiloxane (C) de formule générale :

$$X(Y)_nSi(R)_{3-m}\,Z_m \qquad (VI)$$

et

b) de 0 à 98% en poids d'au moins un monomère (A) lipophile de faible polarité lipophile à insaturation éthylénique, polymérisable par voie radicalaire ;

et/ou

c) de 0 à 98% en poids d'au moins un monomère (B) hydrophile polaire à insaturation éthylénique, copolymérisable avec le ou les monomères du type (A) ;

où :

X désigne un groupe vinylique copolymérisable avec les monomères (A) et (B) ;

Y désigne un groupe de liaison divalent ;

R désigne un hydrogène, un alkyle ou un alcoxy en $C_1$-$C_6$, un aryle $C_6$-$C_{12}$ ;

Z désigne un motif polysiloxane monovalent ayant un poids moléculaire moyen en nombre d'au moins 500 ;

n est 0 ou 1 et m est un entier allant de 1 à 3 ; les pourcentages étant calculés par rapport au poids total des monomères (A), (B) et (C).

Ces polymères ont un poids moléculaire moyen en nombre de préférence allant de 10.000 à 2.000.000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en $C_1$-$C_{24}$ ; le styrène ; les macromères polystyrène ; l'acétate de vinyle ; le propionate de vinyle ; l'alpha-méthylstyrène ; le tertio-butylstyrène ; le butadiène ; le cyclohexadiène ; le cyclohexadiène ; l'éthylène ; le propylène ; le vinyltoluène ; les esters d'acide acrylique ou méthacrylique et de 1,1-dihydroperfluoroalcanols ou de leurs homologues ; les esters d'acide acrylique ou méthacrylique et de oméga-hydrydofluroalcanols ; les esters d'acide acrylique ou méthacrylique et de fluoroalkylsulfoamido-alcools ; les esters d'acide acrylique ou méthacrylique et d'alcools fluoroalkyliques ; les esters d'acide acrylique ou méthacrylique et de fluroéthers d'alcools; ou leurs mélanges. Les monomères (A) préférentiels sont choisis dans le groupe constitué par le méthacrylate de n-butyle, le méthacrylate d'isobutyle, le méthacrylate de stéaryle, l'acrylate de tertio-butyle, le méthacrylate de tertio-butyle, le méthacrylate de 2-éthylhexyle, le méthacrylate de méthyle, le 2-(N-méthyl perfluro-octane sulfonamido)-éthylacrylate ; le 2-(N-butylperflurooctane sulfonamido)-éthyl-acrylate, l'heptadacefluorooctylméthylamino-éthylmethacrylate ou leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butyl-acrylamide, l'acide maléique, l'anhydride maléique et leurs demi-esters, les (méth) acrylates hydroxyalkylés, le chlorure de diallyldiméthylammonium, la vinyl-pyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinyl caprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Les macromères polysiloxane (C) de formule (I) préférentiels sont choisis parmi ceux répondant à la formule générale suivante (VII):

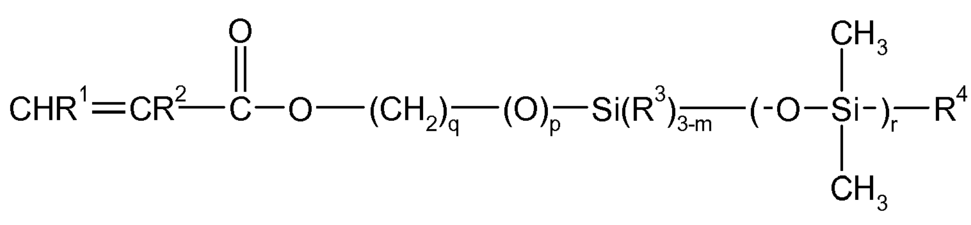

(VII)

dans laquelle :

o $R^1$ est hydrogène ou -COOH et de préférence l'hydrogène ;

o $R^2$ est hydrogène, méthyle ou -$CH_2$COOH (de préférence méthyle) ;

o $R^3$ est alkyle, alcoxy ou alkylamino en $C_1$-$C_6$, aryle en $C_6$-$C_{12}$ ou hydroxyle (de préférence méthyle) ;

o $R^4$ est alkyle, alcoxy ou alkylamino en $C_1$-$C_6$, aryle en $C_6$-$C_{12}$ ou hydroxyle (de préférence méthyle) ;
o q est un entier de 2 à 6 (de préférence 3) ;
o p est 0 ou 1 ;
o r est un nombre entier de 5 à 700 ;
o m est un entier allant de 1 à 3 (de préférence 1) ;

de préférence p=0.

On utilise plus particulièrement les macromères polysiloxanes de formule :

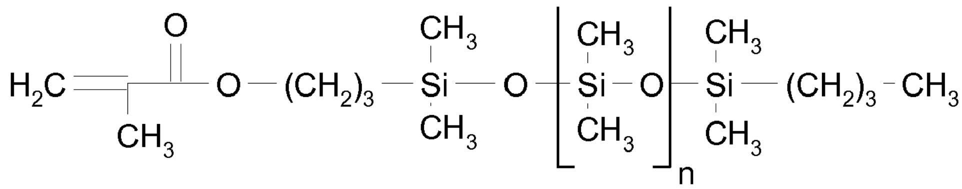

avec n étant un nombre allant de 5 à 700.

Le copolymère à squelette organique non-siliconé greffé par des monomères contenant un polysiloxane peut par exemple avoir la structure suivante :

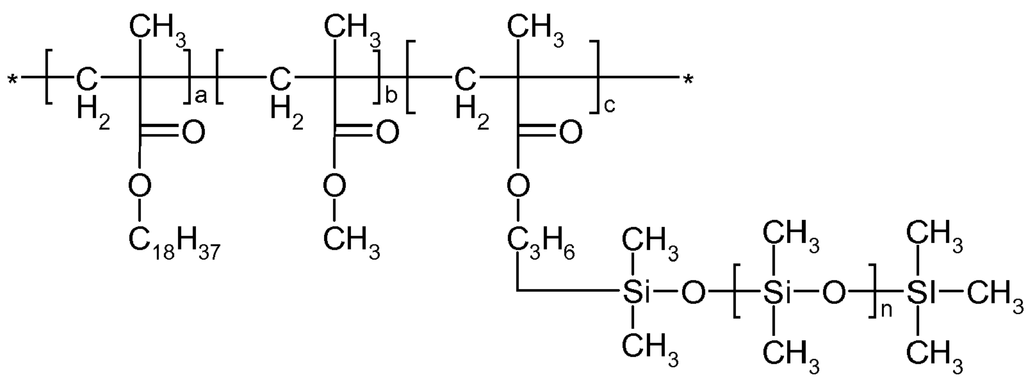

Un tel polymère est commercialisé sous le nom KP 561 commercialisés par Shin Etsu.

Le copolymère à squelette organique non-siliconé greffé par des monomères contenant un polysiloxane peut aussi avoir la structure suivante :

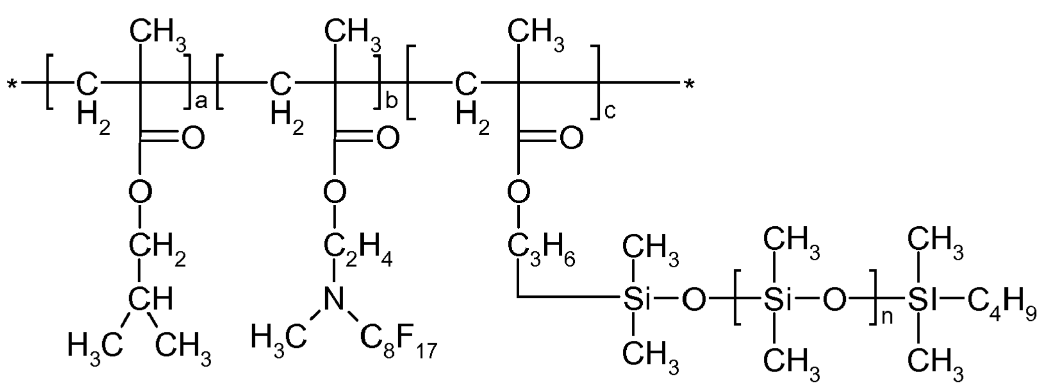

)

Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Un mode particulier de réalisation de l'invention consiste à utiliser un copolymère susceptible d'être obtenu par polymérisation radicalaire à partir du mélange de monomères constitué par :

a) 60% en poids d'acrylate de tertiobutyle ;
b) 20% en poids d'acide acrylique ;
c) 20% en poids de macromère siliconé de formule :

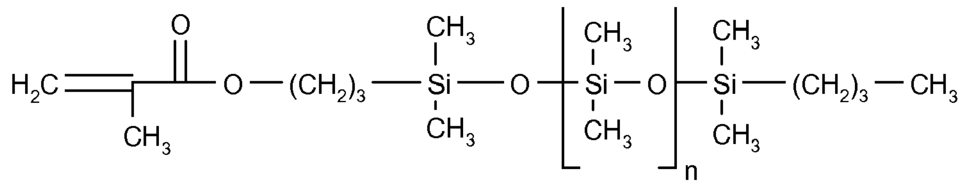

avec n étant un nombre allant de 5 à 700 ; les pourcentages en poids étant calculés par rapport au poids total des monomères.

Un autre mode particulier de réalisation de l'invention consiste à utiliser un copolymère susceptible d'être obtenu par polymérisation radicalaire à partir du mélange de monomères constitué par :

a) 80% en poids d'acrylate de tertiobutyle ;
b) 20% en poids de macromère siliconé de formule :

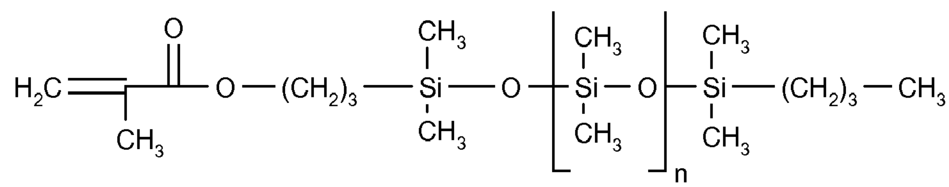

avec n étant un nombre allant de 5 à 700 ; les pourcentages en poids étant calculés par rapport au poids total des monomères.

Une autre famille particulière de polymères siliconés greffés à squelette organique non siliconé convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine. Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, alkyl styrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth) acrylique ; ceux comportant une fonction ester tels que les esters de l'acide (méth) acrylique ; ceux comportant une fonction isocyanate.

Les macromères siliconés sont choisis de préférence parmi les polysiloxanes comportant un groupe fonctionnalisé, au bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale :

$$T\text{-}(CH_2)_s\text{-}Si\text{-}[\text{-}(OSiR^5R^6)_t\text{-}R^7]_y \qquad \text{(VIII)}$$

dans laquelle T est choisi dans le groupe constitué par NH2, NHR', une fonction époxy, OH, SH ; $R^5$, $R^6$, $R^7$ et R', indépendamment, désignent un alkyle en $C_1$-$C_6$, phényle, benzyle, ou alkylphényle en $C_6$-$C_{12}$, hydrogène ; s est un

nombre de 2 à 100 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9000 à 40.000.

Selon la présente invention, le ou les polymères siliconés greffés, à squelette polysiloxanique greffé par des monomères organiques non siliconés comprennent une chaîne principale de silicone (ou polysiloxane ($\equiv$Si-O-)$_n$) sur laquelle se trouve greffé, à l'intérieur de ladite chaîne ainsi qu'éventuellement à l'une au moins de ses extrémités, au moins un groupement organique ne comportant pas de silicone.

Selon un mode particulièrement préféré de réalisation de la présente invention, le polymère siliconé, à squelette polysiloxanique greffé par des monomères organiques non siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un, et de préférence plusieurs, groupements fonctionnels capables de venir réagir sur lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en $C_1$-$C_{18}$ et plus particulièrement en $C_1$-$C_{12}$. Les monomères préférentiels sont choisis dans le groupe constitué par le (méth)acrylate d'isooctyle, le (méth)acrylate d'isononyle, le 2-éthylhexyl (méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non siliconés convenant particulièrement bien à la mise en oeuvre de la présente invention est constituée par les polymères siliconés comportant dans leur structure des motifs de structure (IX ter) et des motifs de structure (IX) et/ou (IX bis) suivants :

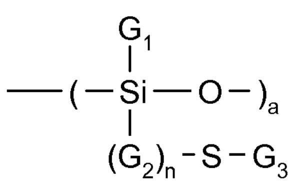

(IX)

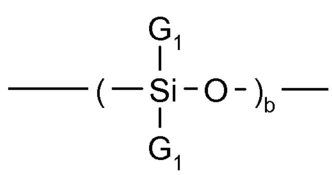

(IX ter)

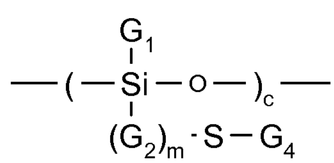

(IX bis)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent représente un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IX) ci-dessus présente au moins l'une, et encore plus préférentiellement l'ensemble, des caractéristiques suivantes :

- les radicaux $G_1$ désignent un radical alkyle, de préférence le radical méthyle ;

- n est non nul, et les radicaux $G_2$ représentent un radical divalent en $C_1$-$C_3$, de préférence un radical propylène ;

- $G_3$ représente un radical polymérique résultant de l'(homo)polymérisation d'au moins un monomère du type acide carboxylique à insaturation éthylénique, de préférence l'acide acrylique et/ou l'acide méthacrylique ;

- $G_4$ représente un radical polymérique résultant de l'(homo)polymérisation d'au moins un monomère du type (méth) acrylate d'alkyle en $C_1$-$C_{10}$, de préférence le (méth)acrylate d'isobutyle ou de méthyle.

Des exemples de polymères siliconés répondant à la formule (VI) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl / methyl siloxane à groupements propyl thio-3 acrylate de methyle / methacrylate de methyle / acide methacrylique ou Polysilicone-8 commercialisé sous le nom VS80 par la société 3M.

D'autres exemples de polymères siliconés répondant à la formule (VI) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl / méthyl siloxane à groupements propyl thio-3-acrylate de méthyle / méthacrylate de méthyle / acide méthacrylique.

La composition de l'invention peut aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ce matériau est capable de retrouver sa forme originelle suite à un étirement. Cet élastomère est formé de chaînes polymériques de haut poids moléculaire dont la mobilité est limitée par un réseau uniforme de points de réticulation. Ces composés ont la propriété d'absorber certains solvants, notamment siliconés, et ainsi de les épaissir, tout en conférant à la composition de très bonnes qualités cosmétiques, notamment d'étalement.

Ces organopolysiloxane peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.

La synthèse de ces organopolysiloxane est décrite dans les brevets suivants :

- US 5266321 de Kobayashi Kose,
- US 4742142 de Toray Silicone,
- US 5654362 de Dow Corning Corp,
- la demande de brevet FR 2 864 784.

Les organopolysiloxanes élastomères utilisés dans la composition peuvent être partiellement ou totalement réticulés. Ils se présentent généralement sous forme de particules. En particulier, les particules d'organopolysiloxane élastomère ont une taille moyenne en nombre allant de 0,1 à 500 $\mu$m, de préférence de 3 à 200 $\mu$m et mieux de 3 à 50 $\mu$m. Ces particules peuvent avoir toute forme et par exemple être sphériques, plates ou amorphes.

L'organopolysiloxane réticulé élastomère peut être obtenu par réaction d'addition réticulation d'un diorganopolysiloxane contenant au moins un atome d'hydrogène lié à un atome de silicium et d'un diorganopolysiloxane ayant au moins deux groupements à insaturation éthylénique liés à des atomes de silicium distincts, notamment en présence de catalyseur platine ; ou par réaction de condensation réticulation, déshydrogénation entre un diorganopolysiloxane à terminaisons hydroxyle et un diorganopolysiloxane contenant au moins un atome d'hydrogène lié à un atome de silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation d'un diorganopolysiloxane (X) contenant au moins un atome d'hydrogène lié à un atome de silicium, et d'un diorganopolysiloxane (XI) ayant au moins deux groupements à insaturation éthylénique liés chacun à un atome de silicium distinct, notamment en présence de catalyseur platine (XII), comme par exemple décrit dans la demande EP-A-295886.

Le composé (X) est en particulier un organopolysiloxane ayant au moins 2 atomes d'hydrogène liés à des atomes de silicium distincts dans chaque molécule. Le composé (X) peut présenter toute structure moléculaire, notamment une structure chaîne linéaire ou chaîne ramifiée ou une structure cyclique. Le composé (X) peut avoir une viscosité à 25 °C allant de 1 à 50 000 centistokes, notamment pour être bien miscible avec le composé (XI).

Les groupes organiques liés aux atomes de silicium du composé (X) peuvent être des groupes alkyles tels que méthyle, éthyle, propyle, butyle, octyle ; des groupes alkyles substitués tels que 2-phényléthyle, 2-phénylpropyle, 3,3,3-trifluoropropyle ; des groupes aryles tels que phényle, tolyle, xylyle ; des groupes aryles substitués tels que phényléthyle ; et des groupes hydrocarbonés monovalents substitués tels qu'un groupe époxy, un groupe ester carboxylate, ou un groupe mercapto. Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (XI) est avantageusement un diorganopolysiloxane ayant au moins deux groupes alkényles inférieurs (par exemple en C2-C4) ; le groupe alkényle inférieur peut être choisi parmi les groupes vinyle, allyle, et propényle. Ces groupements alkényles inférieurs peuvent être situés en toute position de la molécule organopolysiloxane mais sont de préférence situés aux extrémités de la molécule organopolysiloxane.

L'organopolysiloxane (XI) peut avoir une structure à chaîne ramifiée, à chaîne linéaire, cyclique ou en réseau mais la structure en chaîne linéaire est préférée. Le composé (XI) peut avoir une viscosité allant de l'état liquide à l'état de gomme. De préférence, le composé (XI) a une viscosité d'au moins 100 centistokes à 25 °C. Outre les groupes alkényle précités, les autres groupes organiques liés aux atomes de silicium dans le composé (XI) peuvent être des groupes alkyles tels que méthyle, éthyle, propyle, butyle ou octyle ; des groupes alkyles substitués tels que 2-phényléthyle, 2-phénylpropyle ou 3,3,3-trifluoropropyle ; des groupes aryles tels que phényle, tolyle ou xylyle ; des groupes aryles substitués tels que phényléthyle ; et des groupes hydrocarbonés monovalents substitués tels qu'un groupe époxy, un groupe ester carboxylate, ou un groupe mercapto.

Les organopolysiloxanes (XI) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxaneméthylvinylsiloxane à terminaisons triméthylsiloxy, les méthyl(3,3,3-trifluoropropyl) polysiloxane à terminaisons diméthylvinylsiloxy, et les copolymères diméthylsiloxane-méthyl(3,3,3-trifluoropropyl)siloxane à terminaisons diméthylvinylsiloxy. En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (XI) et du nombre d'atomes d'hydrogène liés à des atomes de silicium par molécule du composé (X) est d'au moins 5.

Il est avantageux que le composé (X) soit ajouté en une quantité telle que le rapport moléculaire entre la quantité totale d'atomes d'hydrogène liés à des atomes de silicium dans le composé (X) et la quantité totale de tous les groupements à insaturation éthylénique dans le composé (XI) soit compris dans la gamme de 1,5/1 à 20/1.

Le composé (XII) est le catalyseur de la réaction de réticulation et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support. Le catalyseur (XII) est de préférence ajouté à raison de 0,1 à 1000 parties en poids, mieux de 1 à 100 parties en poids, en tant que métal platine propre pour 1000 parties en poids de la quantité totale des composés (X) et (XI).

L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.

Les particules d'organopolysiloxane réticulé peuvent être véhiculées sous forme de gel constitué d'un organopolysiloxane réticulé inclus dans au moins une huile hydrocarbonée et/ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.

Des organopolysiloxanes réticulés non-émulsionnants sont notamment décrits dans les brevets US4970252, US4987169, US 5412004, US5654362, US5760116, dans la demande JP-A-61-194009.

Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.

Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US5236986, US5412004, US5837793, US5811487.

Comme organopolysiloxanes réticulés émulsionnants, on peut utiliser ceux commercialisés sous les dénominations "KSG-21 ", "KSG-20", "KSG-30", "X-226146" par la société Shin Etsu, et "DC901 0", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US5538793.

De tels élastomères sont vendus sous les dénomination "KSP-100", "KSP-101", "KSP-102", "KSP-103", KSP-104", "KSP-105" par la société Shin Etsu.

D'autres organopolysiloxanes réticulés élastomères sous forme de poudres peuvent être des poudres de silicone hybride fonctionnalisée par des groupes fluoroalkyle, notamment vendues sous la dénomination "KSP-200" par la société Shin Etsu ; ou des poudres de silicones hybrides fonctionnalisées par des groupes phényl, notamment vendues sous la dénomination "KSP-300" par la société Shin Etsu.

D'autres organopolysiloxanes réticulés peuvent se présenter sous forme de dispersions de poudres dans l'eau en présence ou non d'un émulsifiant comme par exemple les composés BY29-119, DC2-1997, EPSX001B, EPSX002B, EPSX004A de Dow Corning.

Lorsqu'il est présent dans la composition de l'invention , le ou les polysiloxanes dont la viscosité est supérieure à 100 cst sont introduits en une quantité généralement comprise entre 0.1 % et 30% en poids, notamment entre 0.1% et 20% en poids et préférentiellement entre 0.1 et 10% en poids.

La composition de l'invention peut aussi comprendre un polymère non siliconé qui permet d'améliorer soit les propriétés intrinsèques de la composition soit le gainage obtenu lors de l'application sur le cheveu, soit les deux.

Un tel polymère peut être choisi parmi les polymères suivants :

- les polymères solubles dans un milieu liquide organique, en particulier les polymères liposolubles;
- les polymères dispersibles dans un milieu solvant organique, en particulier les polymères sous la forme de dispersions non aqueuses de particules de polymères de taille primaire inférieure à 1μm, de préférence des dispersions dans les huiles siliconées ou hydrocarbonées;
- les polymères sous forme de dispersions aqueuses de particules de polymère de taille primaire inférieure à 1μm, souvent appelées « latex » ; dans ce cas, la composition comprend une phase aqueuse ;
- les polymères hydrosolubles ; dans ce cas, la composition comprend une phase aqueuse ou bien le polymère est appliqué en pré ou post-traitement de la composition selon l'invention.

Le polymère utilisable dans la composition peut être anionique, cationique, non-ionique ou amphotère.

La composition peut aussi contenir des charges qui sont généralement des composés substantiellement non colorés solides à température ambiante et pression atmosphérique, et insolubles dans la composition, même lorsque ces ingrédients sont portés à une température supérieure de la température ambiante.

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique). De plus ces particules peuvent être pleines, creuses ou poreuses, enrobées ou non.

Parmi les charges utilisables dans les compositions selon l'invention, on peut notamment citer des charges minérales telles que le talc ; le mica naturel ou synthétique ; le kaolin ; le nitrure de bore, le dioxyde de titane, le carbonate de calcium précipité ; le carbonate et l'hydro-carbonate de magnésium ; l'hydroxyapatite, l'oxyde de cérium, l'oxyde de zirconium.

Avantageusement, la ou les particules inorganiques présentent une taille primaire moyenne en nombre comprise entre 0,1 et 30 μm, de préférence comprise entre 0,2 et 20 μm, et encore plus préférentiellement comprise entre 0,5 et 15 μm. Au sens de la présente invention, on entend par « taille primaire de particule », la dimension maximale qu'il est possible de mesurer entre deux points diamétralement opposés d'une particule individuelle. La taille des particules organiques peut être déterminée par microscopie électronique à transmission ou à partir de la mesure de la surface spécifique par la méthode BET ou à partir d'une granulométrie laser.

De préférence les charges inorganiques utilisées selon l'invention sont le talc, le nitrure de bore, le dioxyde de titane.

Parmi les charges utilisables dans les compositions selon l'invention, on peut notamment citer des charges organiques. Par charge organique, on entend une particule polymérique qui peut être issue de la polymérisation d'un ou de plusieurs monomères. Les polymères constituant ces particules organiques peuvent être réticulés ou non. Les monomères utilisés peuvent être en particulier des esters d'acide méthacrylique ou acrylique, tels que l'acrylate et méthacrylate de méthyle, le chlorure de vinylidène, l'acrylonitrile, le styrène et ses dérivés.

Avantageusement, la ou les particules organiques présentent une taille primaire moyenne en nombre comprise entre 1 et 30 μm, de préférence comprise entre 1 et 20 μm, et encore plus préférentiellement comprise entre 1 et 15 μm.

La ou les particules organiques utilisées dans la composition cosmétique selon l'invention peuvent être choisies parmi les poudres de polyamide, les poudres de polymères acryliques, notamment de polyméthacrylate de méthyle, les poudres de copolymères acryliques, notamment de polyméthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, de polyacrylate/alkylacrylate, les poudres de polystyrène, les poudres de polyéthylène, notamment de polyéthylène/acide acrylique.

A titre représentatif et non limitatif, on peut particulièrement citer en tant que particules organiques selon l'invention :

- les poudres de polyamides (Nylon ®), par exemple celles commercialisées sous les dénominations « ORGASOL ® 4000 » et « ORGASOL ® 2002 UD NAT COS 204 » par la société ATOCHEM,
- les poudres de polymères acryliques, notamment de polyméthacrylate de méthyle, comme par exemple celles commercialisées sous la dénomination « COVABEAD ® LH85 » « COVABEAD ® PMMA »par la société LCW ou celles commercialisées sous la dénomination « MICROPEARL ® MHB » commercialisée par la société MATSUMOTO,
- les poudres de copolymères acryliques, notamment de polyméthacrylate de méthyle/diméthacrylate d'éthylène glycol, comme celles commercialisées sous la dénomination de « DOW CORNING 5640 MICROSPONGE ® SKIN OIL ADSORBER » par la société DOW CORNING, ou celles commercialisés sous la dénomination « GANZPEARL ® GMP-0820 » par la société GANZ CHEMICAL, de polyméthacrylate d'allyle/diméthacrylate d'éthylène glycol, comme celles commercialisées sous la dénomination « POLYPORE ® L200 » ou « POLYPORE ® E200 » commercialisés par la société AMCOL, de copolymère diméthacrylate d'éthylène glycol / méthacrylate de lauryle, comme celles commercialisées par « POLYTRAP ® 6603 » par la société DOW CORNING, de polyacrylate/éthylhexylacrylate comme celles commercialisées sous la dénomination « TECHPOLYMER ® ACX 806C » par la société SEKISUI,
- les poudres de polystyrène / dinvinylbenzène comme celles commercialisées sous la dénomination « TECHPOLYMER ® SBX8 » par la société SEKISUI,
- les poudres de polyéthylène, notamment de polyéthylène/acide acrylique commercialisées sous la dénomination « FLOBEADS ® » par la société SUMITOMO,
- les microsphères de polymères acryliques telles que celles en copolymère d'acrylate réticulé 'POLYTRAP 6603 ADSORBER®' de la société RP SCHERRER
- les poudres de polyuréthane telle que la poudre de copolymère de diisocyanate d'hexaméthylène et de triméthylol hexyl lactone vendue sous la dénomination « PLASTIC POWDER D-400® » par la société Toshiki
- les microcapsules de polymères ou de copolymères d'acrylate ou de méthacrylate de méthyle, ou encore de copolymères de chlorure de vinylidène et d'acrylonitrile, comme l' « EXPANCEL® » de la société EXPANCEL
- les poudres d'organopolysiloxane réticulés élastomères telles que celles vendues sous la dénomination « TREFIL POWDER E-506C » par la société DOW CORNING
- les poudres polyfluorées notamment de polytétrafluoroéthylène, par exemple celle commercialisée sous la dénomination "MP 1400" par la Société DUPONT DE NEMOURS,

De préférence, les particules organiques utilisées dans la composition conforme à l'invention sont choisies parmi les poudres de polyamide et les poudres de polyméthylméthacrylate.

Les compositions selon l'invention peuvent aussi comprendre au moins un agent épaississant des huiles choisi parmi les agents épaississants polymériques, les agents épaississants minéraux et leurs mélanges.

L'agent épaississant polymérique est par exemple un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la phase organique de la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène

et à blocs éthylène/alkylène en C3-C4.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène/propylène.

L'agent épaississant polymérique peut être présent en une teneur allant de 0,1 % à 10 % en poids, par rapport au poids total de la composition, de préférence allant de 0,5 % à 8 % en poids, et plus préférentiellement allant de 1 % à 5 % en poids.

La composition peut également comprendre au moins un agent épaississant minéral des huiles tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite , et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.

- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

L'agent épaississant minéral des huiles peut être présent dans la composition en une teneur allant de 0,1 % à 8 % en poids, par rapport au poids total de la composition, de préférence en une teneur allant de 0,2 % à 6 % en poids, et préférentiellement allant de 0,5 % à 4 % en poids.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des filtres UV, des colloïdes minéraux, des peptisants, des solubilisants, des parfums, des protéines, des vitamines, des propulseurs, les cires oxyéthylénées ou non, les paraffines, les acides gras en C10-C30 tels que l'acide stéarique, l'acide laurique, les amides gras en C10-C30 tel que le diéthanolamide laurique.

Les additifs ci dessus sont en général présents en quantité comprise pour chacun d'eux entre 0,01 et 20 % en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées

La composition selon l'invention peut se présenter sous toute forme appropriée à l'application sur les cheveux, notamment sous forme de crème, de mousse, de stick, de dispersion de vésicules notamment de lipides ioniques ou non, de lotion biphase ou multiphase, de spray d'aérosol, par exemple laques, de poudre, de pâte.

La composition selon l'invention est une composition anhydre, c'est-à-dire une composition contenant moins de 2 % en poids d'eau, voire moins de 0,3 % d'eau, et en particulier exempte d'eau, l'eau présente résultant uniquement de l'eau résiduelle apportée par les ingrédients mélangés.

La composition décrite ci-dessus peut être mise en oeuvre sur cheveux secs ou humides. Les additifs décrits précédemment, lorsqu'ils sont présents, peuvent être appliqués sur les cheveux simultanément avec la composition de l'invention ou séparément. La composition peut être rincée ou non. Il est aussi possible d'effectuer ensuite un lavage des cheveux, ce lavage n'étant pas obligatoire.

## EXEMPLES

**Exemples :**

| | Ex 1 | Ex 2 | Ex 3 | Ex 4 | Ex 5 | Ex 6 |
|---|---|---|---|---|---|---|
| Mélange Polydimethylsiloxane alpha-omega dihydroxyle / Cyclopentadimethylsiloxane (14.7/85.3) commercialisé sous le nom DC1501 Fluid (*) | 20g | 20g | 20g | 20g | 20g | - |
| Silicone linéaire DC200 Fluid 500.000 cst (*) | - | - | - | - | - | 3g |
| BioPSA 7-4400 (*) | 10g | - | 10g | 10g | 10g | 10g |
| BioPSA 7-4500 (*) | - | 10g | - | - | - | - |
| Isododécane | - | - | 30g | - | - | - |
| Silicone linéaire volatile DC200 Fluid 1cst (*) | - | - | - | Qs 100 g | - | - |
| Ethanol | | | | | 20g | - |

(suite)

| | Ex 1 | Ex 2 | Ex 3 | Ex 4 | Ex 5 | Ex 6 |
|---|---|---|---|---|---|---|
| Silicone cyclique volatile DC245 Fluid (*) | Qs 100 g | Qs 100 g | Qs 100 g | - | Qs 100 g | Qs 100 |
| (*) commercialisé par Dow Corning | | | | | | |

0.3g de la composition est appliqué sur une mèche de 1g de cheveux de hauteur de ton 4 propres et humides. Après 15 minutes de pause, la mèche est séchée au sèche-cheveux pendant 2 minutes. On obtient une mèche dont les cheveux sont individualisés et gainés. Ce gainage est rémanent au shampooing.

| | Ex 7 | Ex 8 | Ex 9 | Ex 10 | Ex 11 | Ex 12 |
|---|---|---|---|---|---|---|
| Mélange Polydimethylsiloxane alpha-omega dihydroxyle / Cyclopentadimethylsiloxane (14.7/85.3) commercialisé sous le nom DC1501 Fluid (*) | 20g | 20g | 20g | 20g | 20g | - |
| Silicone linéaire DC200 Fluid 500.000 cst (*) | - | - | - | - | - | 3g |
| BioPSA 7-4400 (*) | 10g | - | 10g | 10g | 10g | 10g |
| BioPSA 7-4500 (*) | - | 10g | - | - | - | - |
| Nacre mica enrobé d'oxyde de fer brun commercialisée par Eckart sous le nom Prestige Bronze | 10g | 10g | 10g | 10g | 10g | 10g |
| Isododécane | - | - | 30g | - | - | 30g |
| Silicone linéaire volatile DC200 Fluid 1cst (*) | - | - | - | Qs 100 g | - | - |
| Ethanol | | | | | 20g | - |
| Silicone cyclique volatile DC245 Fluid (*) | Qs 100 g | Qs 100 g | Qs 100 g | - | Qs 100 g | Qs 100 g |
| (*) commercialisé par Dow Corning | | | | | | |

0.8g de la composition est appliqué sur une mèche de 1g de cheveux propres et humides. Après 15 minutes de pause, la mèche est séchée au sèche-cheveux pendant 2 minutes. On obtient une mèche colorée dont les cheveux sont individualisés et dont la couleur est rémanente au shampooing.

Exemples avec pigments organiques et inorganiques :

| | Ex 13 | Ex 14 | Ex 15 | Ex 16 | Ex 17 | Ex 18 |
|---|---|---|---|---|---|---|
| Mélange Polydimethylsiloxane alpha-omega dihydroxyle / Cyclopentadimethylsiloxane (14.7/85.3) commercialisé sous le nom DC1501 Fluid (*) | 20g | 20g | 20g | 20g | - | - |
| Silicone linéaire DC200 Fluid 500.000 cst (*) | - | - | - | - | 3g | 3g |
| BioPSA 7-4400 (*) | 10g | 10g | 10g | 10g | 10g | 10g |
| Oxyde de fer rouge commercialisé par Sun sous le nom Sunpuro | 10g | | 10g | | 10g | |

(suite)

| | Ex 13 | Ex 14 | Ex 15 | Ex 16 | Ex 17 | Ex 18 |
|---|---|---|---|---|---|---|
| Disperse Red 122 , CI 73915 commercialisé par Sun sous le nom Sunfast Magenta 122 | | 10g | | 10g | | 10g |
| Isododécane | - | - | 30g | 30g | 30g | 30g |
| Silicone cyclique volatile DC245 Fluid (*) | Qs 100 g | Qs 100 g | Qs 100 g | Qs 100 g | Qs 100 g | Qs 100 g |
| (*) commercialisé par Dow Corning | | | | | | |

0.8g de la composition est appliqué sur une mèche de 1g de cheveux propres et humides. Après 15 minutes de pause, la mèche est séchée au sèche-cheveux pendant 2 minutes. On obtient une mèche colorée dont les cheveux sont individualisés et dont la couleur est rémanente au shampooing.

Exemples avec des formules épaissies :

| | Ex 19 | Ex 20 | Ex 21 | Ex 22 | Ex 23 | Ex 24 |
|---|---|---|---|---|---|---|
| Mélange Polydimethylsiloxane alpha-omega dihydroxyle / Cyclopentadimethylsiloxane (14.7/85.3) commercialisé sous le nom DC1501 Fluid (*) | 20g | 20g | 20g | 20g | - | - |
| Silicone linéaire DC200 Fluid 500.000 cst (*) | - | - | - | - | 3g | 3g |
| BioPSA 7-4400 (*) | 10g | 10g | 10g | 10g | 10g | 10g |
| Nacre mica enrobé d'oxyde de fer brun commercialisée par Eckart sous le nom Prestige Bronze | - | 10g | - | 10g | - | 10g |
| Isododécane | - | - | 30g | 30g | 30g | 30g |
| Smectite à 10% dans l'isododécane commercialisée par Elementis sous le nom Bentone Gel ISD v | 25g | 25g | 25g | 25g | 25g | 25g |
| Silicone cyclique volatile DC245 Fluid (*) | Qs 100 | Qs 100 | Qs 100 | Qs 100 | Qs 100 | Qs 100 |
| (*) commercialisé par Dow Corning | | | | | | |

Pour les exemples 19, 21 et 23 :

0.3g de la composition est appliqué sur une mèche de 1g de cheveux de hauteur de ton 4 propres et humides. Après 15 minutes de pause, la mèche est séchée au sèche-cheveux pendant 2 minutes. On obtient une mèche dont les cheveux sont individualisés et gainés. Ce gainage est rémanent au shampooing.

Pour les exemples 20, 22 et 24 :

0.8g de la composition est appliqué sur une mèche de 1g de cheveux propres et humides. Après 15 minutes de pause, la mèche est séchée au sèche-cheveux pendant 2 minutes. On obtient une mèche colorée dont les cheveux sont individualisés et dont la couleur est rémanente au shampooing.

Exemples comparatifs

Les compositions suivantes 25, 25Bis, 26 et 26bis ont été préparées. Les exemples 26 et 26 ont été préparés avec une quantité de BioPSA supérieure à 1 % alors que les compositions 25bis et 26 bis ont été préparées avec une quantité de bioPSA de 1 % exactement.

| | Comp 25 | Comp. 25bis | Comp. 26 | Comp 26bis |
|---|---|---|---|---|
| CYCLOPENTASIL,OXANE (and) DIMETHICONOL vendu par DOW CORNING sous le nom DC 1501 FLUID | 20 | 20 | 20 | 20 |
| BioPSA 7-4400 (*) | 10 | 1 | 10 | 1 |
| Mica nacre recouvert d'oxyde de fer brun vendu par Eckart sous le nom Prestige Bronze | 10 | 10 | - | - |
| Isododécane | - | - | 30 | 30 |
| Smectite à 10% dans isododecane, vendu par Elementis sous le nom Bentone Gel ISD v | 25 | 25 | 25 | 25 |
| silicone Volatile cyclique DC245 Fluid (*) | Qsp 100 | Qsp 100 | Qsp 100 | Qsp 100 |
| (*) Vendu par Dow Corning | | | | |

Chaque composition est appliquée sur des mèches de 1 g ayant une hauteur de ton de 4 (châtain clair). Pour les exemples 25 et 25bis, la quantité de composition appliqué sur les mèches est de 0.8 g. Pour les exemples 26 et 26bis, la quantité de composition appliquée est de 0.3 g.

Les mèches sont ensuite lavées avec un shampooing doux (Ultra doux camomille de Garnier).

Pour les mèches traitées avec les compositions 25 et 25bis, la couleur est évaluée dans le système L*a*b*, avant et après shampoing à l'aide d'un spectrophotomètre Minolta CM-3600d, illuminant D65.

Selon ce système, $L^*$ représente l'intensité. Plus la valeur de $L^*$ est faible, plus la couleur des cheveux est intense. Les coordonnées de chromaticité sont données par les paramètres $a^*$ et $b^*$, $a^*$ représentant l'axe rouge/vert et $b^*$ représentant l'axe jaune/bleu.

ΔE, qui représente la variation de couleur entre les cheveux colorés et les cheveux colorés après le shampooing, est obtenu par la formule suivante :

$$\Delta E = \sqrt{(L^* - L_o^*)^2 + (a^* - a_o^*)^2 + (b^* - b_o^*)^2}$$

dans laquelle L* représente l'intensité et a* et b* la chromaticité après le shampooing et L0* représente l'intensité et a0* et b0* la chromaticité des cheveux avant shampoing. Plus la valeur de ΔE est faible, plus la couleur est résistante au shampooing.

Les résultats obtenus sont reportés dans le tableau suivant :

| | | L* | a* | b* | ΔE |
|---|---|---|---|---|---|
| **Ex 25** | Avant shampooing | 41.99 | 12.27 | 18.83 | - |
| **Ex 25** | Après shampooing | 40.39 | 10.16 | 16.48 | 3.5 |
| **Ex 25bis** | Avant shampooing | 38.26 | 11.11 | 17.33 | - |
| **Ex 25bis** | Après shampooing | 21.9 | 4.31 | 5.9 | 21 |

Ces résultats montrent que lorsque le BioPSA est présent en quantité supérieure à 1 %, on obtient une coloration beaucoup plus résistante au shampoing.

Pour les mèches 26 et 26bis (non colorées), l'évaluation est réalisée de façon qualitative. Au toucher, la mèche 26 après shampoing présente toujours un gainage avec des cheveux présentant plus de corps. Aves les mèches 26bis, le gainage ne semble plus présent après shampoing. Au toucher, la mèche est proche de la mèche avant application de la composition 26 bis.

## Revendications

1. Composition anhydre de traitement des cheveux qui comprend un ou plusieurs copolymères à base de résine de silicone et de silicone fluide, une ou plusieurs silicones volatiles linéaires ou cycliques, et une ou plusieurs polydiméthylsiloxane linéaire non volatile de viscosité supérieure à 5 cSt, la quantité de copolymère étant supérieure à 1 % en poids du poids total de la composition et la composition étant exempte de pigments.

2. Composition anhydre de coloration des cheveux comprenant un ou plusieurs copolymères à base de résine de silicone et de silicone fluide, une ou plusieurs silicones volatiles linéaires ou cycliques, une ou plusieurs polydiméthylsiloxane linéaire non volatile de viscosité supérieure à 5 cSt à 25°C, un pigment colorant, la quantité de copolymère étant supérieure à 1 % en poids du poids total de la composition et la quantité de pigments colorants étant superieure à 5 %.

3. Composition selon la revendication 1 ou 2 dans laquelle le copolymère comprend la résine de silicone à un taux compris entre 40 et 70% et la silicone fluide à un taux compris entre 30 et 60%, avec la somme des pourcentages de résine de silicone et de silicone fluide qui est égal à 100.

4. Composition selon la revendication 1 à 3 dans laquelle la résine de silicone est présente à un taux compris entre 55 et 65% et la silicone fluide est présente à un taux compris entre 35 et 45%.

5. Composition selon l'une quelconque des revendications précédentes dans laquelle le copolymère est présent en une teneur supérieure à 1 % et jusqu'à 40 % en poids, par rapport au poids total de la composition, de préférence allant de 1,5 % à 20 % en poids.

6. Composition selon l'une quelconque des revendications précédentes dans laquelle la silicone volatile est cyclique.

7. Composition selon la revendication 7 dans laquelle la silicone cyclique est choisie parmi l'octaméthylcyclotétrasiloxane, le décaméthylcyclopentasiloxane, le dodécaméthylcyclohexasiloxane, et leurs mélanges, de préférence la décaméthylcyclopentasiloxane.

8. Composition selon l'une quelconque des revendications précédentes dans laquelle la polydiméthylsiloxane linéaire non volatile est choisie parmi les polydiméthylsiloxanes; les alkyldiméthicones; les polyphénylméthylsiloxanes tels que les phényldiméthicones, les phényltriméthicones, et les vinylméthylméthicones; ainsi que les silicones modifiés par des groupements aliphatiques et/ou aromatiques, éventuellement fluorés, ou par des groupements fonctionnels tels que des groupements hydroxyles, thiols et/ou amines.

9. Composition selon la revendication 9 dans laquelle la polydiméthylsiloxane présente une viscosité comprise entre 100 cst et 4.000.000 cSt à 25°C.

10. Composition selon l'une quelconque des revendications 2 à 9 dans laquelle le pigment est un pigment minéral ou une nacre.

11. Composition selon la revendication 10 dans laquelle le pigment est choisi parmi les oxydes de fer ou de chrome, le violet de manganèse, le bleu outremer, l'hydrate de chrome et le bleu ferrique.

12. Composition selon l'une quelconque des revendications précédentes comprenant de plus un solvant additionnel non siliconé, de préférence un hydrocarbure ou un alcool.

13. Composition selon l'une quelconque des revendications précédentes comprenant de plus un agent épaississant, de préférence une argile organomodifiée.

14. Procédé de traitement des fibres kératiniques qui comprend l'application d'une composition telle que définie à l'une quelconque des revendications précédentes, éventuellement suivi après un temps de pose d'un rinçage et/ou lavage.

15. Utilisation d'une composition anhydre comprenant un ou plusieurs copolymères à base de résine de silicone et de silicone fluide et un ou plusieurs pigments colorants pour l'obtention de gainage coloré sur les cheveux, optionnellement un solvant volatile.

**16.** Utilisation selon la revendication 15 dans laquelle le pigment est un pigment minéral, de préférence une nacre.

**17.** Utilisation selon la revendication 22 à 24 dans laquelle la composition comprend de plus une PDMS linéaire non volatile.

**18.** Utilisation d'une composition selon la revendication 1 pour le traitement des cheveux.

Europäisches Patentamt
European Patent Office
Office européen des brevets

# RAPPORT DE RECHERCHE EUROPEENNE

Numéro de la demande
EP 08 16 0223

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | WO 2007/051506 A (UNILEVER PLC [GB]; UNILEVER NV [NL]; LEVER HINDUSTAN LTD [IN]; IVANOVA) 10 mai 2007 (2007-05-10) * page 2, ligne 4 - page 5, ligne 3; revendication 1; exemples 1,2,4 * | 1,3-9, 14,18 | INV. A61K8/58 A61K8/891 A61K8/892 A61Q5/06 A61Q5/12 A61K8/19 |
| X | US 2004/161395 A1 (PATIL ANJALI ABHIMANYU [US] ET AL) 19 août 2004 (2004-08-19) * alinéa [0010] - alinéa [0021] * * alinéa [0029] - alinéa [0050] * * alinéa [0134] - alinéa [0139] * * alinéa [0208] - alinéa [0213]; exemples 2,3 * * alinéa [0234] * | 2-17 | |
| A | EP 0 370 764 A (PROCTER & GAMBLE [US]) 30 mai 1990 (1990-05-30) * page 3, ligne 10 - page 5, ligne 26; exemples VI,VII * | 1-18 | |
| A | WO 2004/084847 A (UNILEVER PLC [GB]; UNILEVER NV [NL]; LEVER HINDUSTAN LTD [IN]; IVANOVA) 7 octobre 2004 (2004-10-07) * page 2, ligne 21 - page 10, ligne 10; exemples * | 1-18 | DOMAINES TECHNIQUES RECHERCHES (IPC) A61K A61Q |
| A | WO 2004/009037 A (E L MANAGEMENT CORP [US]) 29 janvier 2004 (2004-01-29) * page 2, ligne 5 - page 4, ligne 3; exemples 1,2 * | 1-18 | |
| A | FR 2 891 740 A (OREAL [FR]) 13 avril 2007 (2007-04-13) * page 3, ligne 12 - page 9, ligne 7; exemples * | 1-18 | |

Le présent rapport a été établi pour toutes les revendications

| Lieu de la recherche | Date d'achèvement de la recherche | Examinateur |
|---|---|---|
| Munich | 4 novembre 2008 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

4

EPO FORM 1503 03.82 (P04C02)

**ANNEXE AU RAPPORT DE RECHERCHE EUROPEENNE RELATIF A LA DEMANDE DE BREVET EUROPEEN NO.** EP 08 16 0223

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-11-2008

| Document brevet cité au rapport de recherche | | Date de publication | Membre(s) de la famille de brevet(s) | | | Date de publication |
|---|---|---|---|---|---|---|
| WO 2007051506 | A | 10-05-2007 | AR | 056754 | A1 | 24-10-2007 |
| US 2004161395 | A1 | 19-08-2004 | WO | 2004073626 | A2 | 02-09-2004 |
| EP 0370764 | A | 30-05-1990 | AT | 117539 | T | 15-02-1995 |
| | | | CA | 2003393 | A1 | 21-05-1990 |
| | | | DE | 68920834 | D1 | 09-03-1995 |
| | | | DE | 68920834 | T2 | 28-09-1995 |
| | | | ES | 2066865 | T3 | 16-03-1995 |
| | | | PT | 92352 | A | 31-05-1990 |
| | | | US | 4983383 | A | 08-01-1991 |
| WO 2004084847 | A | 07-10-2004 | JP | 2006521303 | T | 21-09-2006 |
| | | | US | 2006280693 | A1 | 14-12-2006 |
| WO 2004009037 | A | 29-01-2004 | AU | 2003256967 | A1 | 09-02-2004 |
| | | | CA | 2497396 | A1 | 29-01-2004 |
| | | | EP | 1545434 | A2 | 29-06-2005 |
| FR 2891740 | A | 13-04-2007 | BR | PI0604435 | A | 28-08-2007 |
| | | | CN | 1977800 | A | 13-06-2007 |
| | | | EP | 1787630 | A2 | 23-05-2007 |
| | | | JP | 2007106764 | A | 26-04-2007 |
| | | | KR | 20070038924 | A | 11-04-2007 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2833489
- WO 03026596 A
- WO 2004073626 A
- WO 2007051505 A
- WO 2007051506 A
- US 5162410 A
- CA 711756
- FR 2679771
- EP 1184426 A
- US 6225198 B
- US 5990479 A
- US 4578266 A
- US 4693935 A
- US 4728571 A
- US 4972037 A
- EP 0412704 A
- EP 0412707 A
- EP 0640105 A
- WO 9500578 A
- US 5266321 A
- US 4742142 A
- US 5654362 A
- FR 2864784
- EP 295886 A
- US 4970252 A
- US 4987169 A
- US 5412004 A
- US 5760116 A
- JP 61194009 A
- US 5236986 A
- US 5837793 A
- US 5811487 A
- US 5538793 A
- US 2002005562 A
- US 5221534 A


### Littérature non-brevet citée dans la description


- Silicone Pressure Sensitive Adhesive. **SOBIESKI ; TANGNEY.** Handbook of Pressure Sensitive Adhesive Technology. Von Nostrand Reinhold
- **DABBOUSSI B.O. et al.** CdSe)ZnS core-shell quantum dots : synthesis and characterisation of a size series of highly luminescent nanocristallites. *Journal of phisical chemistry B,* 1997, vol. 101, 9463-9475
- **PENG ; XIAOGANG et al.** Epitaxial Growth of highly Luminescent CdSe/CdS core/shell nanocrystals with photostability and electronic accessibility. *Journal of the American Chemical Society,* vol. 119 (30), 7019-7029
- *Cosmetics and Toiletries,* Février 1990, vol. 105, 53-64
- **S. CAILLÈRE ; S. HÉNIN ; M. RAUTUREAU.** Minéralogie des argiles. Masson, 1982
- Silica silylate. CTFA. 1995
- Silica diméthyl silylate. CTFA. 1995